# EUROPEAN PATENT APPLICATION

(11) **EP 2 415 742 A1**
(43) Date of publication of application: **08.02.2012**
(21) Application number: 10758915.2
(22) Date of filing: 30.03.2010
(51) Int. Cl.: C07C 29/149, B01J 31/22, C07C 31/20, C07C 33/22, C07C 67/31, C07C 69/76, C07F 15/00, C07B 61/00

(54) **METHOD FOR PRODUCING ALCOHOL COMPOUND AND CATALYST THEREFOR**

(30) Priority: 31.03.2009 JP 2009084833
(71) Applicant: Sumitomo Chemical Company, Limited, Tokyo 104-8260 (JP)
(72) Inventor: AOYAGI, Yasutaka, Oita-shi Oita 870-0832 (JP); SOUDA, Hiroshi, Oita-shi Oita 870-0025 (JP); HAGIYA, Koji, Ibaraki-shi Osaka 567-0833 (JP)
(74) Representative: Vossius & Partner
(86) International application number: PCT/JP2010/056130
(87) International publication number: WO 2010/114151

(57) **Abstract**

A method for producing an alcohol compound, wherein a carboxylic acid ester compound is reduced with hydrogen in the presence of a ruthenium complex which is obtained by reacting a pyridine compound having at least one optionally substituted amino group with a ruthenium compound.

## Description

### TECHNICAL FIELD

The present invention relates to a method for producing an alcohol compound and a catalyst therefor.

### BACKGROUND ART

A method for producing an alcohol compound, wherein a carboxylic acid ester compound is reduced with hydrogen in the presence of a ruthenium catalyst, is known (See, e.g., Japanese Unexamined Patent Application Publication No. 2001-247499, WO 2008/120475, Japanese Unexamined Patent Application Publication No. 2004-300131, Japanese Unexamined Patent Application Publication (Translation of PCT Application) Nos. 2008-537946 and 2008-538352, and Angew. Chem. Int. Ed., 45, 1113 (2006)).

### DISCLOSURE OF THE INVENTION

The present invention relates to a novel method for producing an alcohol compound and a novel ruthenium complex which is used for the production method.

That is, the present application relates to the following inventions.
[1] A method for producing an alcohol compound, wherein a carboxylic acid ester compound is reduced with hydrogen in the presence of a ruthenium complex which is obtained by reacting a pyridine compound having at least one optionally substituted amino group with a ruthenium compound.
[2] The production method as described in [1], wherein in the pyridine compound, a pyridine ring is linked to the optionally substituted amino group through a linking group.
[3] The production method as described in [2], wherein the linking group is an optionally substituted alkylene group.
[4] The production method as described in any one of [1] to [3], wherein the pyridine compound has two optionally substituted amino groups.
[5] The production method as described in any one of [1] to [4], wherein the optionally substituted amino group is an amino group, an alkylamino group having 1 to 4 carbon atoms, a dialkylamino group having 2 to 8 carbon atoms, an arylamino group having 6 to 10 carbon atoms, a diarylamino group having 12 to 20 carbon atoms, or a cyclic amine group having 2 to 8 carbon atoms.
[6] The production method as described in any one of [1] to [5], wherein the pyridine compound is represented by the following formula 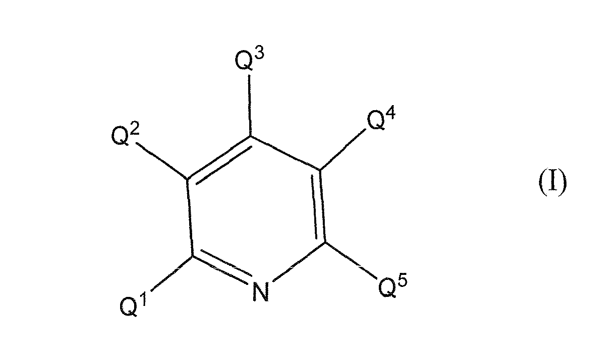 wherein Q¹, Q², Q³, Q⁴ and Q⁵ independently represent a hydrogen atom, an optionally substituted alkyl group, an optionally substituted aryl group, an optionally substituted alkoxy group, an amino group, or an optionally substituted aminoalkyl group, or each of Q¹ and Q², Q² and Q³, Q³ and Q⁴, and Q⁴ and Q⁵ may together represent a divalent group, with the proviso that at least one of Q¹, Q², Q³, Q⁴ and Q⁵ represents an amino group or an optionally substituted aminoalkyl group.
[7] The production method as described in any one of [1] to [5], wherein the pyridine compound has a structure represented by the formula (A) 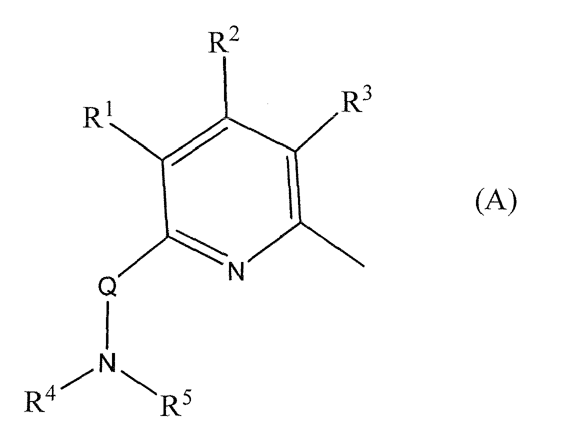 wherein R¹ represents a hydrogen atom, an optionally substituted alkyl group, an optionally substituted aryl group, or an optionally substituted alkoxy group, or with R² or Q, represents a divalent substituent; R² represents a hydrogen atom, an optionally substituted alkyl group, an optionally substituted aryl group, or an optionally substituted alkoxy group, or with R¹, represents a divalent substituent; R³ represents a hydrogen atom, an optionally substituted alkyl group, an optionally substituted aryl group, or an optionally substituted alkoxy group;
   R⁴ and R⁵ each independently represent a hydrogen atom, an optionally substituted alkyl group, or an optionally substituted aryl group, or R⁴ and R⁵ bind to each other, together with a nitrogen atom, to form a cyclic amino group having 2 to 8 carbon atoms; Q represents an optionally substituted alkylene group or a single bond, or with R¹, represents a divalent substituent).
[8] The production method as described in any one of [1] to [7], wherein the ruthenium compound is at least one member selected from the group consisting of a compound comprised of halogens and ruthenium, an aromatic compound-coordinated ruthenium dihalide dimer, a diene-coordinated ruthenium dihalide polymer, and a tris(triphenylphosphine)ruthenium compound.
[9] The production method as described in any one of [1] to [8], wherein the carboxylic acid ester compound is a carboxylic acid ester compound having an aliphatic hydrocarbon group, a carboxylic acid ester compound having an aromatic hydrocarbon group, or a cyclic carboxylic acid ester compound.
[10] The production method as described in any one of [1] to [8], wherein the carboxylic acid ester compound is a compound represented by the formula (6) 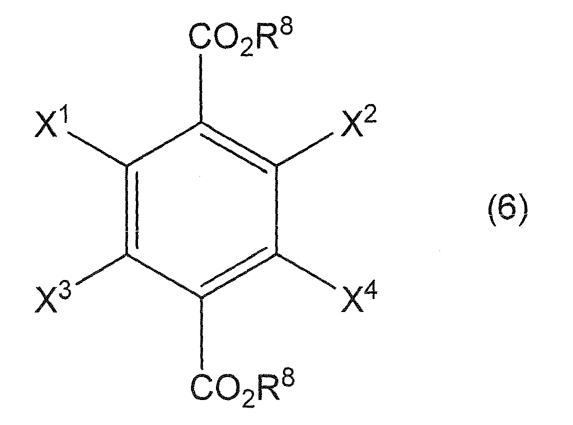 wherein R⁸ represents an alkyl group having 1 to 6 carbon atoms; and X¹, X², X³ and X⁴ each independently represent a hydrogen atom or a halogen atom, with the proviso that at least one of X¹, X², X³ and X⁴ is a halogen atom.
[11] The production method as described in any one of [1] to [10], wherein the ruthenium complex is used in the range of 0.001 to 0.2 mol per mol of ester structure in the carboxylic acid ester compound.
[12] The production method as described in any one of [1] to [11], wherein the carboxylic acid ester compound is reduced with hydrogen in the presence of a base.
[13] A ruthenium complex which is obtained by reacting a pyridine compound having at least one optionally substituted amino group with a ruthenium compound.
[14] A composition for producing an alcohol compound containing a ruthenium complex which is obtained by reacting a pyridine compound having at least one optionally substituted amino group with a ruthenium compound.

### MODE FOR CARRYING OUT THE INVENTION

The present invention will now be described in detail.

First, a pyridine compound having at least one optionally substituted amino group will be described.

The above pyridine compound generally has an optionally substituted pyridine ring and an optionally substituted amino group.

Such a pyridine ring may be directly bound to the optionally substituted amino group, and may be linked to the amino group through a linking group.

The above pyridine compound may have two or more each of pyridine rings and amino groups. When the above pyridine compound has two each of pyridine rings and amino groups, a dimer may be formed by binding the two pyridine rings to each other or the two amino groups to each other through a single bond.

Substituents of the pyridine rings include optionally substituted alkyl groups, optionally substituted aryl groups, optionally substituted alkoxy groups and the like.

The above optionally substituted alkyl groups may be linear, branched and cyclic chains, and preferred are alkyl groups having 1 to 20 carbon atoms.

Among the above optionally substituted alkyl groups, unsubstituted alkyl groups include linear or branched alkyl groups having 1 to 20 carbon atoms such as a methyl group, an ethyl group, a propyl group, an isopropyl group, a butyl group, an isobutyl group, a sec-butyl group, a tert-butyl group, a pentyl group and a decyl group; and cycloalkyl groups having 3 to 10 carbon atoms such as a cyclopropyl group, a 2,2-dimethylcyclopropyl group, a cyclopentyl group, a cyclohexyl group and a menthyl group.

Among the above optionally substituted alkyl groups, substituted alkyl groups have optionally substituted aryl groups, optionally substituted alkoxy groups, optionally substituted aryloxy groups, and halogen atoms as substituents. Examples of the above optionally substituted aryl groups include aryl groups having 6 to 10 carbon atoms such as a phenyl group and a naphthyl group; (C1-C4 alkyl)-substituted aryl groups such as a 2-methylphenyl group and a 4-methylphenyl group; halogen-substituted aryl groups such as a 4-chlorophenyl group; (C1-C4 alkoxy) -substituted aryl groups such as a 4-methoxyphenyl group. Examples of the above optionally substituted alkoxy groups include alkoxy groups having 1 to 6 carbon atoms such as a methoxy group, an ethoxy group, a propoxy group, an isopropoxy group, a butoxy group, an isobutoxy group, a sec-butoxy group, and a tert-butoxy group; haloalkoxy groups having 1 to 6 carbon atoms such as a fluoromethoxy group and a trifluoromethoxy group; a benzyloxy group; (C1-C4 alkyl)-substituted benzyloxy groups such as a 4-methylbenzyloxy group; a 3-phenoxybenzyloxy group; (C3-C10 cycloalkyl)-substituted benzyloxy groups such as a cyclopentylbenzyloxy group; (C1-C4 alkoxy)-substituted C1-C4 alkoxy groups such as a methoxymethoxy group, an ethoxymethoxy group, and a methoxyethoxy group.

Examples of the above optionally substituted aryloxy groups include a phenoxy group; [(C1-C4 alkyl)-substituted aryl]oxy groups such as a 2-methylphenoxy group and a 4-methylphenoxy group; [(C1-C4 alkoxy)-substituted aryl]oxy groups such as a 4-methoxyphenoxy group; and (phenoxy-substituted aryl) oxy groups such as a 3-phenoxyphenoxy group.

Examples of the above halogen atoms include a fluorine atom and a chlorine atom.

Among the above optionally substituted alkyl groups, substituted alkyl groups include haloalkyl groups such as a fluoromethyl group and a trifluoromethyl group; (C1-C4 alkoxy)-substituted C1-C4 alkyl groups such as a methoxymethyl group, an ethoxymethyl group and a methoxyethyl group; aryl-substituted alkyl groups such as a benzyl group; (halogen-substituted aryl)C1-C4 alkyl groups such as a 4-fluorobenzyl group; (alkyl-substituted aryl)C1-C4 alkyl groups such as a 4-methylbenzyl group; and (phenoxy-substituted) C1-C4 alkyl groups such as a phenoxymethyl group.

In each substituent exemplified in this specification, C1-C4 and C3-C10 represent 1 to 4 carbon atoms and 3 to 10 carbon atoms, respectively.

Among the above substituents of the pyridine rings, optionally substituted aryl groups are preferably optionally substituted aryl groups having 6 to 10 carbon atoms. Among the above optionally substituted aryl groups, unsubstituted aryl groups include aryl groups having 6 to 10 carbon atoms, specifically, a phenyl group and a naphthyl group.

Among the above optionally substituted aryl groups, substituted aryl groups have as substituents, for example, the optionally substituted alkyl groups described above, the optionally substituted alkoxy groups described above and the halogen atoms described above.

Among the above optionally substituted aryl groups, substituted aryl groups include (C1-C4 alkyl)-substituted aryl groups such as a 2-methylphenyl group and a 4-methylphenyl group; halogen-substituted aryl groups such as a 4-chlorophenyl group; and (C1-C4 alkoxy)-substituted aryl groups such as a 4-methoxyphenyl group.

The optionally substituted alkoxy groups are represented by -OR (wherein R is an optionally substituted alkyl group). In R, the optionally substituted alkyl group includes the alkyl groups as exemplified above.

Substituents of the above pyridine rings are preferably alkyl groups having 1 to 6 carbon atoms, aryl groups having 6 to 10 carbon atoms, a benzyl group, and (C1-C4 alkyl) -substituted benzyl groups.

In the above pyridine rings, two carbon atoms adjacent to each other may be substituted with a divalent substituent. Examples of such a divalent substituent include alkylene groups having 1 to 4 carbon atoms such as a methylene group, an ethylene group, a propylene group, and a butylene group.

When the pyridine ring is linked to the above amino group through a linking group, a substituent of the pyridine ring and the linking group may bind, together with the pyridine ring, to form a bicyclic ring. Such bicyclic rings include a quinoline ring, a cyclopenteno pyridine ring, a cyclohexenopyridine ring and the like.

The optionally substituted amino group is a group in which a hydrogen atom(s) of the amino group may be substituted with a substituent(s). When two hydrogen atoms of the amino group are substituted, it may be a cyclic amino group.

Examples of substituents which the above amino groups can have include optionally substituted alkyl groups and optionally substituted aryl groups. Such alkyl groups include a methyl group, an ethyl group, a propyl group, an isopropyl group, a butyl group, an isobutyl group, a sec-butyl group, a tert-butyl group and the like. Such aryl groups include the aryl groups exemplified above.

The optionally substituted amino groups include an amino group, alkylamino groups and arylamino groups. The alkylamino groups include alkylamino groups having 1 to 4 carbon atoms such as a methylamino group and an ethyl amino group; and dialkylamino groups having 2 to 8 carbon atoms such as a dimethylamino group and a diethylamino group. The arylamino groups include arylamino groups having 6 to 10 carbon atoms such as a phenylamino group; and diarylamino groups having 12 to 20 carbon atoms such as a diphenylamino group.

Examples of the cyclic amino groups include cyclic amino groups having 2 to 8 carbon atoms such as a 1-aziridinyl group, a 1-azetidinyl group, a 1-pyrrolidinyl group and a piperidino group.

Specifically, the optionally substituted amino groups are represented by the following formula

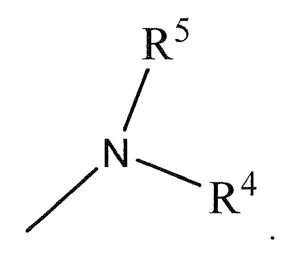

In the above formula, R⁴ and R⁵ each independently represent a hydrogen atom, an optionally substituted alkyl group or an optionally substituted aryl group, or R⁴ and R⁵ bind to each other, together with a nitrogen atom, to form a cyclic amino group having 2 to 8 carbon atoms.

The above pyridine compound preferably has two optionally substituted amino groups. When the above pyridine compound has two substituted amino groups, a dimer may be formed by binding substituents of each amino group to each other.

In the above pyridine compound, linking groups include optionally substituted alkylene groups and the like.

Examples of the alkylene groups in the above linking groups include alkylene groups having 1 to 6 carbon atoms such as a methylene group, an ethylene group, a propylene group, an isopropylene group, a butylene group, an isobutylene group, a pentylene group, an isopentylene group, and a hexylene group.

Substituents which the above alkylene groups can have include optionally substituted aryl groups having 6 to 10 carbon atoms; optionally substituted alkoxy groups having 1 to 6 carbon atoms; optionally substituted aryloxy groups having 6 to 10 carbon atoms; and halogen atoms.

Substituents which the above alkylene groups can have preferably include aryl groups having 6 to 10 carbon atoms such as a phenyl group and a naphthyl group; substituted aryl groups having 6 to 10 carbon atoms such as a 4-methylphenyl group and a 4-methoxyphenyl group; alkoxy groups having 1 to 4 carbon atoms such as a methoxy group, an ethoxy group, a propoxy group, an isopropoxy group, a butoxy group, an isobutoxy group, a sec-butoxy group and a tert-butoxy group; substituted alkoxy groups having 1 to 4 carbon atoms such as a trifluoromethoxy group, a benzyloxy group, a 4-methylbenzyloxy group, a 4-methoxybenzyloxy group and a 3-phenxoybenzyloxy group; optionally substituted aryloxy groups having 6 to 10 carbon atoms such as a phenoxy group, a 2-methylphenoxy group, a 4-methylphenoxy group, a 4-methoxyphenoxy group and a 3-phenoxyphenoxy group; halogen atoms such as a fluorine atom and a chlorine atom; and the like.

The substituted alkylene groups include a fluoromethylene group, a methoxymethylene group, a phenylmethylene group, a fluoroethylene group, a methoxyethylene group, 2-methoxypropylene group and the like.

The above pyridine compound is preferred that an optionally substituted amino group be linked to the carbon atom at 2-position in the pyridine ring through a linking group, or the amino groups be linked to each carbon atom at 2- and 5-potisions in the pyridine ring through linking groups.

When the above pyridine compound has one pyridine ring, it is preferred that positions other than the sites to which amino groups are bound or linked not be substituted.

When the above pyridine compound has two pyridine rings, each pyridine ring is preferably bound through a single bond.

The above pyridine compound is, for example, represented by the following formula 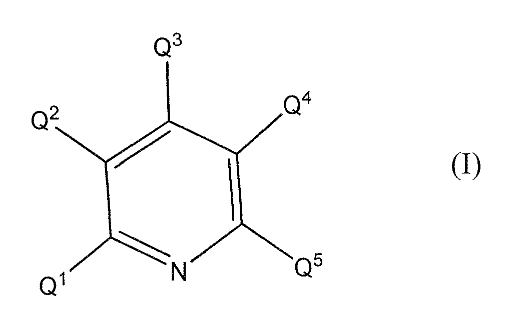 (wherein Q¹, Q², Q³, Q⁴ and Q⁵ independently represent a hydrogen atom, an optionally substituted alkyl group, an optionally substituted aryl group, an optionally substituted alkoxy group, an optionally substituted amino group or an optionally substituted aminoalkyl group, or each of Q¹ and Q², Q² and Q³, Q³ and Q⁴, and Q⁴ and Q⁵ may together represent a divalent group, with the proviso that at least one of Q¹, Q², Q³, Q⁴ and Q⁵ represents an amino group or an optionally substituted aminoalkyl group).

In the formula (I), an optionally substituted alkyl group, an optionally substituted aryl group, an optionally substituted alkoxy group and an optionally substituted amino group each include the groups exemplified as the substituents of the above pyridine rings. The above optionally substituted aminoalkyl groups include aminoalkyl groups having 1 to 4 carbon atoms, (C1-C4 alkylamino)(C1-C4 alkyl)groups, and [di(C1-C4 alkylamino)](C1-C4 alkyl)groups. Specific examples of the above optionally substituted aminoalkyl groups include an aminomethyl group, a methylaminomethyl group, a (dimethylamino)methyl group, an ethylaminomethyl group, (diethylamino)methyl group, a (dipropylamino)methyl group, and an aminoethyl group.

In the formula (I), examples of the divalent substituent include alkylene groups having 2 to 6 carbon atoms, alkenylene groups having 3 to 6 carbon atoms, and dienediyl groups having 3 to 6 carbon atoms.

The pyridine compound preferably has a group represented by the formula (A)

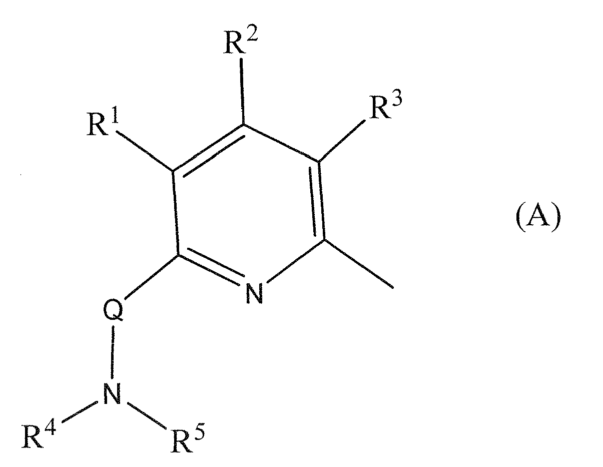

In the formula, R¹ represents a hydrogen atom, an optionally substituted alkyl group, an optionally substituted aryl group or an optionally substituted alkoxy group, or with R² or Q, represents a divalent substituent.
R² represents a hydrogen atom, an optionally substituted alkyl group, an optionally substituted aryl group or an optionally substituted alkoxy group, or with R¹, represents a divalent substituent. R³ represents a hydrogen atom, an optionally substituted alkyl group, an optionally substituted aryl group or an optionally substituted alkoxy group.

In R¹, R² and R³, an optionally substituted alkyl group, an optionally substituted aryl group and an optionally substituted alkoxy group each include the groups exemplified as the substituents of pyridine rings.

In R¹ and R², the divalent substituent includes the groups exemplified as the substituents of pyridine rings.

R⁴ and R⁵ each independently represent a hydrogen atom, an optionally substituted alkyl group or an optionally substituted aryl group, or R⁴ and R⁵ bind to each other, together with a nitrogen atom, to form a cyclic amino group having 2 to 8 carbon atoms.

In R⁴ and R⁵, an optionally substituted alkyl group and an optionally substituted aryl group each include the groups exemplified as the substituents of amino groups.

The cyclic amino group formed from R⁴, R⁵ and a nitrogen atom includes the groups exemplified as the substituents of amino groups.

Q represents an optionally substituted alkylene group, or with R¹, represents a divalent substituent.

The optionally substituted alkylene group includes the groups exemplified as the above linking groups.

Examples of the divalent substituent represented by Q and R¹ include alkylene groups having 2 to 6 carbon atoms, alkenylene groups having 3 to 6 carbon atoms, and dienediyl groups having 3 to 6 carbon atoms.

Examples of the pyridine compound include a 2-amino(C1-C4 alkyl)pyridine, a 2-(C1-C4 alkylamino) (C1-C4 alkyl)pyridine, a 2-[di(C1-C4 alkyl)amino](C1-C4 alkyl)pyridine, a 2-[(phenylamino)methyl]pyridine, a 3-amino(C1-C4 alkyl)pyridine, a 3-(C1-C4 alkylamino) (C1-C4 alkyl)pyridine, a 3-[di(C1-C4 alkyl)amino](C1-C4 alkyl)pyridine, a 3-[(phenylamino)methyl]pyridine, a 4-amino(C1-C4 alkyl)pyridine, a 4-(C1-C4 alkylamino) (C1-C4 alkyl)pyridine, a 4-[di(C1-C4 alkyl)amino](C1-C4 alkyl)pyridine, a 4-[(phenylamino)methyl]pyridine, a 2,5-bis[amino(C1-C4 alkyl)]pyridine, a 2,5-bis[(C1-C4 alkylamino)(C1-C4 alkyl)]pyridine, a 2,5-bis[[di(C1-C4 alkyl)amino](C1-C4 alkyl)]pyridine, a 2,6-bis[amino(C1-C4 alkyl)]pyridine, a 2,6-bis[(C1-C4 alkylamino)(C1-C4 alkyl)]pyridine, a 2,6-bis[di(C1-C4 alkyl)amino(C1-C4 alkyl)]pyridine, and a 2,6-bis[(phenylamino)methyl]pyridine.

A preferred pyridine compound includes, for example, a compound represented by the formula (3) 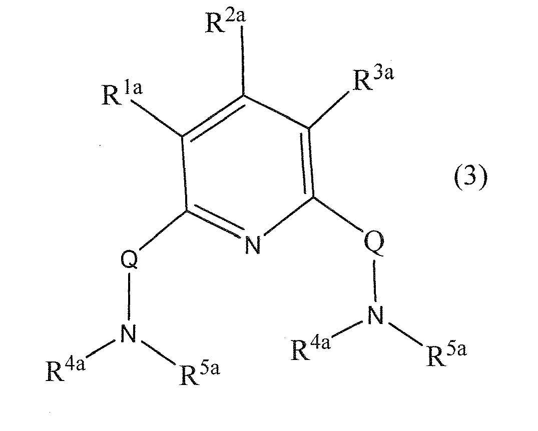 (hereinafter this compound is abbreviated as Pyridine Compound (3)).

In the formula, R^{1a} and R^{2a} each independently represent a hydrogen atom, an optionally substituted alkyl group, an optionally substituted aryl group or an optionally substituted alkoxy group, or R^{1a} and R^{2a} together represent a divalent substituent.
R^{3a} represents a hydrogen atom, an optionally substituted alkyl group, an optionally substituted aryl group or an optionally substituted alkoxy group.

In R^{1a}, R^{2a} and R^{3a}, an optionally substituted alkyl group, an optionally substituted aryl group and an optionally substituted alkoxy group each include the groups exemplified as the substituents of pyridine rings.

R^{4a} and R^{5a} each independently represent a hydrogen atom, an alkyl group having 1 to 4 carbon atoms or an aryl group having 6 to 10 carbon atoms, or R^{4a} and R^{5a} bind to each other, together with a nitrogen atom, to represent a cyclic amino group having 2 to 8 carbon atoms.

In R^{4a} and R^{5a}, an alkyl group having 1 to 4 carbon atoms includes a methyl group, an ethyl group, a propyl group and a butyl group, and an aryl group having 6 to 10 carbon atoms includes a phenyl group and a naphthyl group.

Examples of such Pyridine Compound (3) include
2,6-bis(aminomethyl)pyridine,
2,6-bis[(methylamino)methyl]pyridine,
2,6-bis[(dimethylamino)methyl]pyridine,
2,6-bis[(diethylamino)methyl]pyridine, α2, α2, α6, α6-tetramethyl-2,6-bis(aminomethyl)pyridine,
2,6-bis(aminomethyl)-4-methoxypyridine,
2,6-bis(aminomethyl)-4-dimethylaminopyridine,
2,6-bis(aminomethyl)-4-methylpyridine,
2,6-bis(aminoethyl)pyridine,
2,6-bis[(methylamino)methyl]pyridine,
2,6-bis[(dimethylamino)ethyl]pyridine,
2,6-bis(aminopropyl)pyridine,
2,6-bis[(methylamino)propyl]pyridine,
2,6-bis[(dimethylamino)propyl]pyridine,
2,6-bis[(diphenylamino)methyl]pyridine,
2,6-bis[(phenylamino)methyl]pyridine, and the like. These Pyridine Compounds (3) may form a salt with a hydrogen halide such as hydrogen chloride or hydrogen bromide, and a mineral acid such as sulfuric acid or phosphoric acid.

A preferred pyridine compound further includes a compound having a bicyclic ring such as a quinoline ring, a cyclopentenopyridine ring or a cyclohexenopyridine ring.

Examples of such compound having a bicyclic ring include N,N'-[bis(8-quinolyl)]ethane-1,2-diamine and 6,6'-bis(aminomethyl)-1,2'-bipyridyl.

A pyridine compound may be one which is commercially available or which is produced according to a known method.

Methods for producing pyridine compounds, for example, include a method in which a pyridine having a group represented by X-Q- as a substituent (wherein X represents a leaving group; and Q has the same meaning as described above) is reacted with an alkylamine to convert X into an alkylamino group, a method in which a pyridine aldehyde is reacted with an alkylamine hydrochloride, and the like.
The above leaving group includes a halogen, a hydroxyl group and the like.

Specifically, Pyridine Compound (3) can be produced by methods shown in Route 1 to 3 below. Route 1, Route 2 and Route 3 are described in Inorganic Chemistry, 36, 4812 (1997); Tetorahedron, 62, 9973 (2006); and Liebigs Ann. Chem., 537 (1978), respectively. 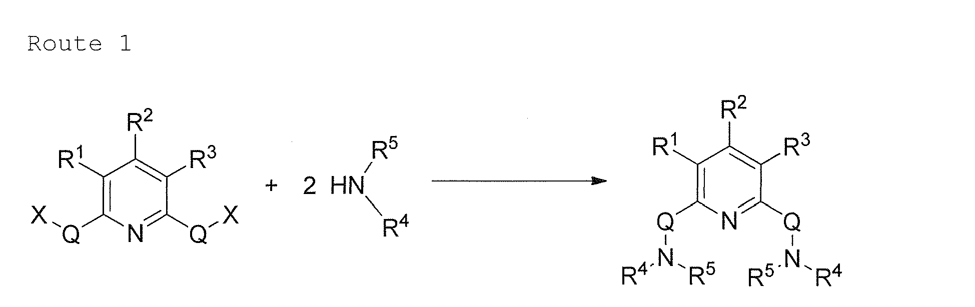 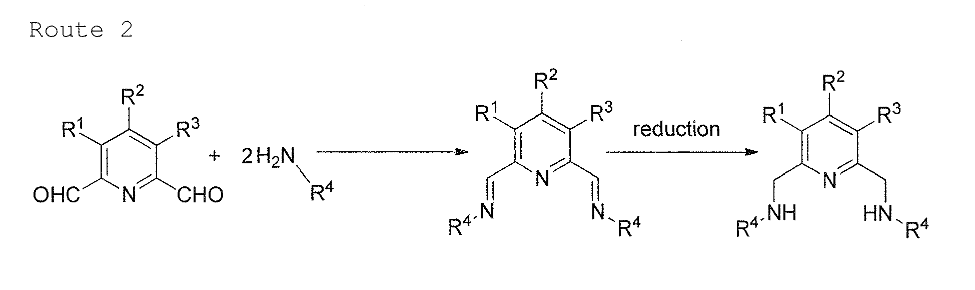 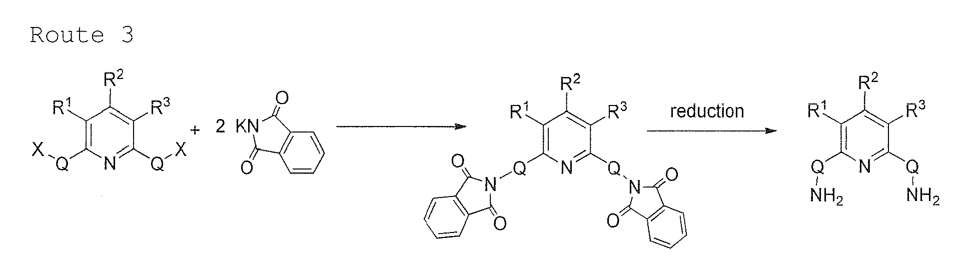 (wherein R¹, R², R³, R⁴, R⁵ and Q each represent the same meanings as described above; and X represents a chlorine atom, a bromine atom or an iodine atom)

Next, a ruthenium complex which is obtained from the reaction of a pyridine compound and a ruthenium compound (hereinafter abbreviated as ruthenium complex), and a composition for producing an alcohol compound will be described. The above ruthenium complex can be produced at a low cost since it is obtained from the reaction of the above pyridine compound and a ruthenium compound. The above ruthenium complex and the above composition for producing an alcohol compound are useful as catalysts for reduction of a carboxylic acid ester compound.

Examples of the ruthenium compound include compounds comprised of halogens and ruthenium such as ruthenium(III) chloride and ruthenium(III) bromide; aromatic compound-coordinated ruthenium dihalide dimers such as (p-cymene)ruthenium dichloride dimer, (benzene)ruthenium dichloride dimer, (mesitylene)ruthenium dichloride dimer, (hexamethylbenzene)ruthenium dichloride dimer, and (p-cymene)ruthenium dibromide dimer; diene-coordinated ruthenium dihalide polymers such as ruthenium 1,5-cyclooctadiene dichloride polymer, and ruthenium dinorbornadiene dichloride polymer; tris(triphenylphosphine)ruthenium compounds such as tris(triphenylphosphine)ruthenium dichloride, tris(triphenylphosphine)ruthenium dibromide, tris(triphenylphosphine)ruthenium hydrochloride, and carbonyl(dihydride)tris(triphenylphosphine)ruthenium; and preferred are tris(triphenylphosphine)ruthenium compounds.

These ruthenium compounds may be hydrates. The ruthenium compounds may be used individually, or two or more may also be used simultaneously.

A ruthenium compound may be one which is commercially available or which is synthesized by any known method.

The amount of a ruthenium compound, which is converted into the amount of ruthenium atoms, can be used in the range of generally 0.5 to 5 mol, preferably 0.6 to 2 mol, and more preferably 0.8 to 1.5 mol per mol of a pyridine compound.

In the above ruthenium compounds, the amount of ruthenium atoms is determined by known means such as element analysis using ICP emission spectroscopy.

A synthesis of a ruthenium complex is generally performed in the presence of an organic solvent.

Examples of such organic solvent include ether solvents such as methyl tert-butyl ether, tetrahydrofuran, dimethoxyethane, and diglyme; nitrile solvents such as acetonitrile and propionitrile; aromatic hydrocarbon solvents such as toluene and xylene; halogenated hydrocarbon solvents such as dichloromethane, chloroform and 1,2-dichloroethane; and the like, and preferred are ether solvents, aromatic hydrocarbon solvents and halogenated hydrocarbon solvents.

The amount to be used of the organic solvent is not particularly restricted, and is generally 1 part by weight or more and 500 parts by weight or less, and preferably 300 parts by weight or less per part by weight of the pyridine compound in view of productivity.

In the synthesis of a ruthenium complex, the order of mixing a pyridine compound, a ruthenium compound and the like is not restricted.

The synthesis of a ruthenium complex can be performed by, for example, mixing a pyridine compound with a ruthenium compound in the presence of an organic solvent under reaction temperature conditions.

The reaction temperature at the time of producing a ruthenium complex is generally in the range of -20° to 100°C, and preferably 0° to 40°C. The progress of the reaction can be confirmed by general analytical means such as high performance liquid chromatography, thin layer chromatography, NMR and IR.

In the obtained reaction mixture, a ruthenium complex is contained. The reaction mixture, per se, or the reaction mixture on which isolation and purification are performed can be used for reduction described below.

The above isolation includes, for example, washing, separation, crystallization and condensation. The purification includes recrystallization, column chromatography and the like. It is preferred that the above ruthenium complex be isolated.

In the present invention, a ruthenium complex which is isolated from the reaction mixture is preferred.

The composition for producing an alcohol compound of the present invention includes the reaction mixtures and powders or solutions containing ruthenium complexes isolated from the reaction mixtures. Since the composition contains a ruthenium complex, it can be suitably used for producing a carboxylic acid ester compound described below.

Next, a method for producing an alcohol compound, wherein a carboxylic acid ester compound is reduced with hydrogen in the presence of a ruthenium complex, will be described.

The above carboxylic acid ester compound is an organic compound having one or more esters. The above carboxylic acid ester compound may be a monoester and a compound having multiple esters such as a diester. The above compound having multiple esters includes an oxalic acid diester, a malonic acid diester, a phthalic acid diester, a maleic acid diester, a glutaric acid diester and an adipic acid diester.

The above carboxylic acid ester compound includes an carboxylic acid ester compound having an aliphatic hydrocarbon group (hereinafter this compound is referred to as "aliphatic hydrocarbon-containing carboxylic acid ester"), a carboxylic acid ester compound having an aromatic hydrocarbon group (hereinafter this compound is referred to as "aromatic hydrocarbon-containing carboxylic acid ester"), and a cyclic carboxylic acid ester compound.

In the above carboxylic acid ester compounds, the above aliphatic hydrocarbon group includes optionally substituted alkyl groups and optionally substituted alkenyl groups.

Examples of such alkyl groups include linear, branched or cyclic alkyl groups having 1 to 20 carbon atoms such as a methyl group, an ethyl group, a propyl group, an isopropyl group, a butyl group, an isobutyl group, a sec-butyl group, a tert-butyl group, a pentyl group, a decyl group, a cyclopropyl group, a 2,2-dimethylcyclopropyl group, a cyclopentyl group, a cyclohexyl group and a methyl group. Examples of substituents of the alkyl groups include halogen atoms such as a fluorine atom; alkoxy groups having 1 to 4 carbon atoms such as a methoxy group and an ethoxy group; an amino group; a hydroxyl group; carbonyloxyalkyl groups having 2 to 5 carbon atoms. Specific examples of the substituted alkyl groups include substituted alkyl groups having 1 to 20 carbon atoms such as a fluoromethyl group, a trifluoromethyl group, a methoxymethyl group, an ethoxymethyl group, a methoxyethyl group, a hydroxymethyl group and an aminomethyl group.

Examples of such alkenyl groups include linear, branched or cyclic alkenyl groups having 2 to 12 carbon atoms such as an ethenyl group, a 1-propenyl group, a 1-methylethenyl group, a 1-methyl-2-propenyl group, a 1-butenyl group, a 2-butenyl group, a 3-butenyl group, a 1-methyl-1-propenyl group, a 2-methyl-1-propenyl group, a 1-pentenyl group, a 2-pentenyl group, a 1-hexenyl group, a 1-decenyl group, a 1-cyclopentenyl group and a 1-cyclohexenyl group. Examples of substituents of the alkenyl groups include halogen atoms such as a fluorine atom, a chlorine atom and a bromine atom; alkoxy groups such as a methoxy group and an ethoxy group; an amino group; a hydroxyl group; and the like. Specific examples of the substituted alkenyl groups include a 3-fluoro-1-propenyl group and a 3-methoxy-1-propenyl group.

Specific examples of the above aliphatic hydrocarbon-containing carboxylic acid ester include ethyl acetate, methyl propionate, isopropyl butanoate, octyl pentanoate, benzyl hexanoate, pentyl heptanoate, methyl octanoate, benzyl cyclohexanecarboxylate, benzyl pivalate, butyl tert-butyl acetate, ethyl acrylate, ethyl 3,3-dimethyl-2-(2-methyl-1-propenyl)cyclopropanecarboxylate, benzyl
3,3-dimethyl-2-(2-methyl-1-propenyl)cyclopropanecarboxylate, methyl 3,3-dimethyl-2-(1-propenyl)cyclopropanecarboxylate, heptyl pyruvate, dimethyl oxalate, diethyl malonate, dipropyl glutarate, and dibutyl adipate.

The above aliphatic hydrocarbon-containing carboxylic acid ester may be one which is commercially available or which is produced by a known method.

In the above carboxylic acid ester compounds, the above aromatic hydrocarbon group includes optionally substituted aryl groups.

Examples of such aryl groups include aryl groups having 6 to 10 carbon atoms such as a phenyl group, a 1-naphthyl group and a 2-naphthyl group. Examples of substituents of the aryl groups include the optionally substituted alkyl groups; the optionally substituted alkenyl groups; halogen atoms such as a fluorine atom, a chlorine atom and a bromine atom; alkoxy groups such as a methoxy group and an ethoxy group; an amino group; a hydroxyl group; carbonyloxyalkyl groups; and the like. Specific examples of the substituted aryl groups include a 2-methylphenyl group, a 4-chlorophenyl group, a 4-methylphenyl group, a 4-methoxyphenyl group, a 4-aminophenyl group, a 4-hydroxyphenyl group, a 3-phenoxy-1-butenyl group and a styryl group.

The above aromatic hydrocarbon-containing carboxylic acid ester includes, for example, a compound represented by the formula (6) 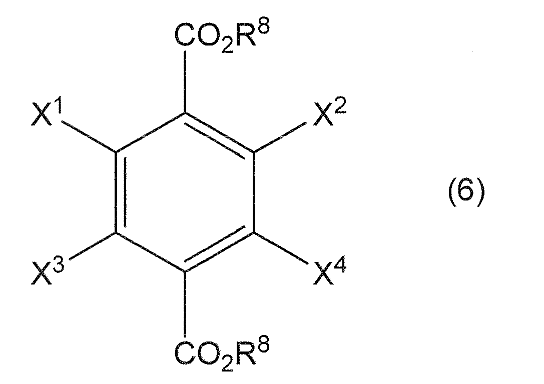 (wherein R⁸ represents an alkyl group having 1 to 6 carbon atoms; and X¹, X², X³ and X⁴ each independently represent a hydrogen atom or a halogen atom, with the proviso that at least one of X¹, X², X³ and X⁴ is a halogen atom).

The alkyl group having 1 to 6 carbon atoms represented by R⁸ includes a methyl group, an ethyl group, a propyl group, an isopropyl group, a butyl group, an isobutyl group, a sec-butyl group, a tert-butyl group and a pentyl group.

Specific examples of the above aromatic hydrocarbon-containing carboxylic acid ester include ethyl cinnamate, (1-octyl)cinnamate, benzyl phenylacetate, methyl benzoate, isopropyl benzoate, methyl 2-fluorobenzoate, benzyl
2-fluorobenzoate, methyl 2-chlorobenzoate, ethyl
2-bromobenzoate, propyl 3-fluorobenzoate, butyl
3-chlorobenzoate, pentyl 3-bromobenzote, methyl
4-fluorobenzoate, methyl 4-aminobenzoate, methyl
4-bromobenzoate, benzyl 2,4-difluorobenzoate, ethyl
2,4-dichlorobenzoate, methyl 3,5-difluorobenzoate, methyl
2,3,5,6-tetrafluorobenzoate, methyl
4-methyl-2,3,5,6-tetrafluorobenzoate, methyl
3-phenoxybenzoate, methyl 4-methyl benzoate, methyl
3-trifluoromethyl benzoate, methyl 2-methoxybenzoate, methyl
4-phenylbutyrate, methyl 3-(4-hydroxyphenyl)propanoate,
1-methoxycarbonylnaphthalene, dimethyl phthalate, diethyl isophthalate, dimethyl terephthalate, dimethyl
3,4,5,6-tetrafluorophthalate, diethyl
2,4,5,6-tetrafluoroisophthalate, dimethyl terephthalate, dimethyl 2-fluoroterephthalate, dimethyl 2-chloroterephthalate, dimethyl 2,5-difluoroterephthalate, dimethyl
2,6-difluoroterephthalate, dimethyl 2,3-difluoroterephthalate, dimethyl 2,5-dichloroterephthalate, dimethyl
2,6-dichloroterephthalate, dimethyl 2,3-dichloroterephthalate, dimethyl 2,3,5-trifluoroterephthalate, dimethyl
2,3,5-trichloroterephthalate, dimethyl
2,3,5,6-tetrafluoroterephthalate, diethyl
2,3,5,6-tetrafluoroterephthalate, dipropyl
2,3,5,6-tetrafluoroterephthalate, diisopropyl
2,3,5,6-tetrafluoroterephthalate, dibutyl
2,3,5,6-tetrafluoroterephthalate, di tert-butyl
2,3,5,6-tetrafluoroterephthalate, dimethyl
2,3,5,6-tetrachloroterephthalate, diethyl
2,3,5,6-tetrachloroterephthalate, dipropyl
2,3,5,6-tetrachloroterephthalate, diisopropyl
2,3,5,6-tetrachloroterephthalate, dibutyl
2,3,5,6-tetrachloroterephthalate, di tert-butyl
2,3,5,6-tetrachloroterephthalate, dipentyl
2,3,5,6-tetrachloroterephthalate, dihexyl
2,3,5,6-tetrachloroterephthalate, and dimethyl
2,3,5-trifluoro-6-chloroterephthalate.

The above aromatic hydrocarbon-containing carboxylic acid ester can be produced according to, for example, a method reacting an acid halide in which a corresponding n atom; an alkoxy group such as a methoxy group or an ethoxy group; an acid halide such as a phenyl group or a naphthyl group, and an alcohol (See, e.g., Japanese Examined Patent Application Publication No. H04-66220).

In the above carboxylic acid ester compounds, a cyclic carboxylic acid ester compound includes optionally substituted lactones.

Such lactones are preferably 4- to 22- membered rings. It is preferred that the above lactone be a ring structure containing an optionally substituted alkylene group having 2 to 20 carbon atoms. Such alkylene groups include an ethylene group, a propylene group, an isopropylene group, a butylene group, a pentylene group, a hexylene group, a heptalene group, an octalene group and a decylene group. Examples of substituents of the alkylene groups include halogearyl groups such as a fluorine atom; aryloxy groups such as a phenoxyl group, a 1-naphthyloxy group and a 2-naphthyloxy group; alkenyl groups such as an ethenyl group, a 1-propenyl group, a 1-methylethenyl group, a 1-butenyl group, a 1-methyl-1-propenyl group, a 2-methyl-1-propenyl group, a 1-pentenyl group, a 1-hexenyl group and a 1-decenyl group; an amino group; a hydroxyl group; and the like. Specific examples of substituted alkylene groups include a fluoroethylene group, a methoxymethylene group, a 2-hydroxypropylene group, a 2-aminobutylene group, a 2-phenylmethylbutylene group and the like.

The above cyclic carboxylic acid ester compounds include β-propiolactone, γ-butyrolactone, δ-valerolactone, ε-caprolactone, β-methyl-ε-caprolactone, γ-methyl-ε-caprolactone, heptanolactone, octanolactone, nonanolactone, decanolactone and the like. A cyclic carboxylic acid ester compound may be one which is commercially available or which is produced by a known method.

Commercially available hydrogen gas is generally used as hydrogen for the above reduction. The hydrogen pressure is generally in the range of 0.1 to 5 MPa.

The amount to be used of a ruthenium complex is the range of preferably 0.001 to 0.2 mol, and more preferably 0.01 to 0.2 mol per mol of ester in the above carboxylic acid ester compound.

When a ruthenium atom is coordinated by halide ions in a ruthenium complex, the above reduction is preferably carried out in the presence of a base. Upon being carried out in the presence of a base, the catalytic activity of such a ruthenium complex is improved since halide ions in the complex are removed.

Examples of such a base include alkali metal hydroxides such as lithium hydroxide, sodium hydroxide and potassium hydroxide; alkaline-earth metal hydroxides such as magnesium hydroxide and calcium hydroxide; alkali metal alkoxides such as sodium methoxide, sodium ethoxide and potassium tert-butoxide; and alkali metal hydrides such as lithium hydride, sodium hydride and potassium hydride; and preferred are alkali metal hydroxides.

The amount to be used of the base is the range of generally 1 to 100 mol, preferably 1 to 10 mol, and more preferably 1 to 5 mol per mol of halide ion coordinating to a ruthenium complex.

The composition of the above ruthenium complex and the amount of halide ions in the complex can be determined by a known method such as element analysis using ICP emission spectroscopy.

When a hydrogen atom-coordinated ruthenium compound such as carbonyl(dihydride)tris(triphenylphosphine)ruthenium is used, the reduction is preferably carried out in the absence of a base.

The above reduction is generally carried out in the presence of an organic solvent. Examples of such an organic solvent include ether solvents such as methyl tert-butyl ether, tetrahydrofuran, dimethoxyethane and diglyme; alcohol solvents such as methanol, ethanol and isopropanol; aromatic hydrocarbon solvents such as toluene and xylene; and aliphatic hydrocarbon solvents such as n-hexane, n-heptane and cyclohexane; and preferred are ether solvents and aromatic hydrocarbon solvents.

The amount of the organic solvent is not particularly restricted, and generally 1 part by weight or more and 100 parts by weight or less, and preferably 50 parts by weight or less per part by weight of the carboxylic acid ester compound in view of productivity and the like.

The above reduction is, for example, carried out by mixing a carboxylic acid ester compound, a ruthenium complex and, if needed, an organic solvent and/or a base, and then replacing the inside of a device for the above reduction with hydrogen to adjust hydrogen pressure and reaction temperature.

In the above reduction, the reaction temperature is the range of generally 0° to 200°C, and preferably 50° to 180°C. In the above reduction, the reaction pressure is the range of generally 0.1 to 5 MPa, and preferably 0.5 to 5 MPa. The progress of the reaction can be confirmed by general analytical means such as gas chromatography, high performance liquid chromatography, thin-layer chromatography, NMR, and IR.

After the reaction termination, an alcohol compound which is a product is contained in the obtained reaction mixture. An objective alcohol compound can be isolated by performing general isolation such as washing, separation, crystallization and condensation on the reaction mixture.

When an insoluble matter such as a ruthenium complex is deposited in the reaction mixture, the above isolation may be performed after removing the insoluble matter by filtration and the like, if needed.

An organic solvent which is not miscible with water may be used for the above separation treatment, if needed. Also, an isolated alcohol compound may be purified by general purifying means such as distillation and column chromatography.

The organic solvent which is not miscible with water herein includes aromatic hydrocarbon solvents such as toluene, xylene and chlorobenzene; aliphatic hydrocarbon solvents such as pentane, hexane and heptane; halogenated hydrocarbon solvents such as dichloromethane, dichloroethane and chloroform; ether solvents such as diethylether and methyl tert-butyl ether; ester solvents such as ethyl acetate; and the like.

An alcohol compound obtained by the above reduction is a compound having -CH₂OH. The -CH₂OH is a group formed by reducing an ester in a carboxylic acid ester compound with hydrogen.

When aliphatic hydrocarbon-containing carboxylic acid esters are used as a carboxylic acid ester compound, the following alcohol compounds are, for example, obtained.

Ethanol, 1-propanol, isopropanol, 1-butanol, isobutanol, tert-butanol, 1-pentanol, cyclopentanol, 1-hexanol, 1-heptanol, 1-octanol, 1-nonanol, 1-decanol, allyl alcohol, ethylene glycol, 1,3-propanediol, 1,4-butanediol, 1,6-hexanediol, 3,3-dimethyl-2-(2-methyl-1-propenyl)cyclopropanemethanol, and 3,3-dimethyl-2-(1-propenyl)cyclopropanemethanol.

When aromatic hydrocarbon-containing carboxylic acid esters are used as a carboxylic acid ester compound, the following alcohol compounds are, for example, obtained.

Benzyl alcohol, 2-fluorobenzyl alcohol, 3-fluorobenzyl alcohol, 4-fluorobenzyl alcohol, 2-chlorobenzyl alcohol,
4-chlorobenzyl alcohol, 4-aminobenzyl alcohol, 4-methoxybenzyl alcohol, 4-methyl-2,3,5,6-tetrafluorobenzyl alcohol,
2,3,5,6-tetrafluorobenzyl alcohol, 2-phenyl-1-ethanol,
4-phenyl-1-butanol, 3-(4-hydroxyphenyl)-1-propanol,
1-naphthylmethanol, 1,2-benzenedimethanol,
1,3-benzenedimethanol, 1,4-benzenedimethanol,
3,4,5,6-tetrafluoro-1,2-benzenedimethanol, and
2,4,5,6-tetrafluoro-1,3-benzenedimethanol.

When cyclic carboxylic acid esters are used as a carboxylic acid ester compound, the following alcohol compounds are, for example, obtained.

1,3-Propanediol, 1,4-butanediol, 1,5-pentanediol, 1,6-hexanediol, 3-methyl-1,6-hexanediol, 4-methyl-1,6-hexanediol, 1,7-heptanediol, 1,8-octanediol, 1,9-nonanediol, and 1,10-decanediol.

When compounds having multiple esters are used as a carboxylic acid ester compound to perform the reaction, alcohol compounds whose one or more esters are reduced with hydrogen are obtained.

When halogen-substituted terephthalic acid diesters are, for example, used as a carboxylic acid ester compound, as obtained alcohol compounds, a compound represented by the formula (7) 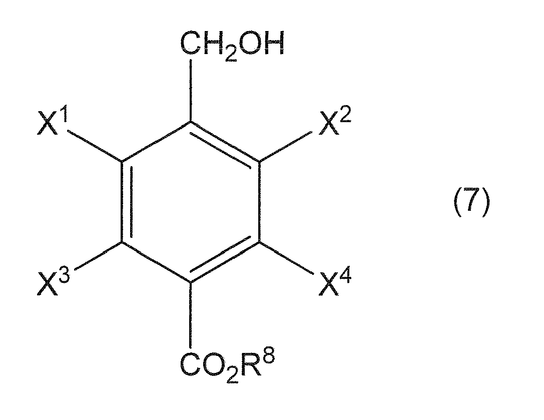 (wherein R⁸ represents an alkyl group having 1 to 6 carbon atoms; X¹, X², X³ and X⁴ each independently represent a hydrogen atom or a halogen atom, with the proviso that at least one of X¹, X², X³ and X⁴ is a halogen atom) (hereinafter may be referred to as Compound (7)) and/or a compound represented by the formula (8) 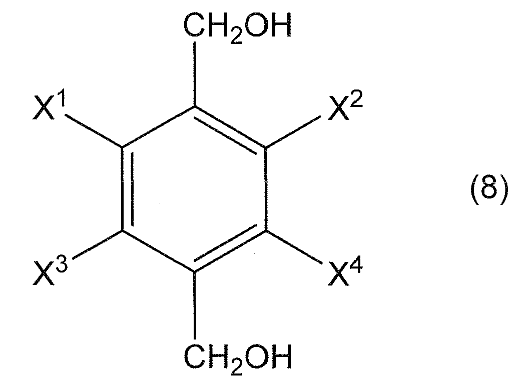 (wherein X¹, X², X³ and X⁴ each represent the same meanings as described above) (hereinafter may be referred to as Compound (8)) can be obtained.

Examples of Compound (7) include methyl
2-fluoro-(4-hydroxymethyl)benzoate, ethyl
2-chloro-(4-hydroxymethyl)benzoate, methyl
2,5-difluoro-(4-hydroxymethyl)benzoate, propyl
2,6-difluoro-(4-hydroxymethyl)benzoate, methyl
2,3-difluoro-(4-hydroxymethyl)benzoate, methyl
2,5-dichloro(4-hydroxymethyl)benzoate, methyl
2,6-dichloro-(4-hydroxymethyl)benzoate, methyl
2,3-dichloro-(4-hydroxymethyl)benzoate, methyl
2,3,5-trifluoro-(4-hydroxymethyl)benzoate, methyl
2,3,5-trichloro-(4-hydroxymethyl)benzoate, methyl
2,3,5,6-tetrafluoro-(4-hydroxymethyl)benzoate, ethyl
2,3,5,6-tetrafluoro-(4-hydroxymethyl)benzoate, propyl
2,3,5,6-tetrafluoro-(4-hydroxymethyl)benzoate, butyl
2,3,5,6-tetrafluoro-(4-hydroxymethyl)benzoate, methyl
2,3,5,6-tetrachloro-(4-hydroxymethyl)benzoate, and methyl
2,3,5-trifluoro-6-chloro-(4-hydroxymethyl)benzoate.

Examples of Compound (8) include
2-fluoro-1,4-benzenedimethanol,
2-chloro-1,4-benzenedimethanol,
2,5-difluoro-1,4-benzenedimethanol,
2,6-difluoro-1,4-benzenedimethanol,
2,3-difluoro-1,4-benzenedimethanol,
2,5-dichloro-1,4-benzenedimethanol,
2,6-dichloro-1,4-benzenedimethanol,
2,3,-dichloro-1,4-benzenedimethanol,
2,3,5-trifluoro-1,4-benzenedimethanol,
2,3,5-trichloro-1,4-benezenedimethanol,
2,3,5,6-tetrafluorobenenzenedimethanol,
2,3,5,6-tetrachlorobenzenedimethanol, and
2,3,5-trifluoro-6-chlorobenzenedimethanol.

### EXAMPLES

The present invention will be described in more detail by way of examples, but the present invention is not limited by the examples.

The melting point was measured using an automatic melting point measuring apparatus, METTLER FP62, manufactured by Mettler-Toledo International Inc.

NMR was measured using an FT-NMR apparatus, DPX 300, manufactured by Bruker Japan Co., Ltd.

Gas chromatography was measured using GC-17A, manufactured by Shimadzu Corporation.

### Example 1

Into a 50 mL flask equipped with a reflux condenser, 200 mg of tris (triphenylphosphine) ruthenium dichloride and 50 mL of dichloromethane were charged. While stirring the obtained mixture at room temperature (25°C), to the mixture, a mixture of 36 mg of 2,6-bis(aminomethyl)pyridine and 5 mL of dichloromethane was added, and immediately the color of the mixture was changed from bluish purple into reddish purple, and deposition of a crystal was found. The obtained mixture, per se, was stirred at room temperature for 30 minutes, and then the crystal was obtained by filtering the reaction mixture, followed by drying to yield 150 mg of a reddish purple powder containing a ruthenium complex. The melting point of the reddish purple powder was 190° to 193°C (decomposition).

### Example 2

Into a 100 mL autoclave equipped with a glass cylinder, 23 mg of the reddish purple powder obtained from Example 1, 24 mg of potassium hydroxide, 200 mg of methyl benzoate and 10 g of tetrahydrofuran were charged. The inside of the autoclave was replaced with nitrogen and then replaced with hydrogen, followed by increasing the hydrogen pressure to 1.0 MPa. Then, upon increasing the temperature to 100°C, the inner pressure became 1.3 MPa. Upon stirring the contents of the autoclave at 100°C for 16 hours, the inner pressure became 1.2 MPa.
When the contents of the autoclave were cooled to room temperature and analyzed by gas chromatography (internal standard method), the yield of benzyl alcohol was 24%. Also, methyl benzoate recovery was 50%. Besides, benzyl benzoate, which was supposed to be produced from benzyl alcohol and methyl benzoate, was produced as a by-product in 10%.

### Example 3

Into a 100 mL autoclave equipped with a glass cylinder, 26 mg of the reddish purple powder obtained from Example 1, and 13 mg of potassium hydroxide, 200 mg of dimethyl 2,3,5,6-tetrafluoroterephthalate and 10 g of toluene were charged. The inside of the autoclave were replaced with nitrogen and then replaced with hydrogen, followed by increasing the hydrogen pressure to 1.0 MPa. Then, upon increasing the temperature to 100°C, the inner pressure became 1.6 MPa. Upon stirring the contents of the autoclave at 170°C for 4 hours, the inner pressure became 1.5 MPa. When the contents of the autoclave were cooled to room temperature and analyzed by gas chromatography (internal standard method), the yield of methyl 2,3,5,6-tetrafluoro-4-hydroxymethyl benzoate was 25%. Also, dimethyl 2,3,5,6-tetrafluoroterephthalate recovery was 64%. Besides, methyl 2,3,5,6-tetrafluorobenzoate was produced as a by-product in 12%.

### Example 4

Into a 50 mL flask equipped with a reflux condenser, 200 mg of tris (triphenylphosphine) ruthenium dichloride and 50 mL of dichloromethane were charged. While stirring the obtained mixture at room temperature, to the mixture, a mixture of 66 mg of N,N'-[bis(8-quinolyl)]ethane-1,2-diamine and 5 mL of dichloromethane was added, and immediately the color of the mixture was changed from bluish purple into black purple, and deposition of a crystal was found. The obtained mixture, per se, was stirred at room temperature for 30 minutes, and then the crystal was obtained by filtering the reaction mixture, followed by drying to yield 150 mg of a black powder containing a ruthenium complex. The melting point of the black powder was 195° to 198°C (decomposition).

### Example 5

Into a 100 mL autoclave equipped with a glass cylinder, 22 mg of the black powder obtained from Example 4, 14 mg of potassium hydroxide, 200 mg of dimethyl
2,3,5,6-tetrafluoroterephthalate and 10 g of toluene were charged. The inside of the autoclave was replaced with nitrogen and then replaced with hydrogen, followed by increasing the hydrogen pressure to 1.0 MPa. Then, upon increasing the temperature to 100°C, the inner pressure became 1.6 MPa. Upon stirring the contents of the autoclave at 170°C for 4 hours, the inner pressure became 1.5 MPa. When the contents of the autoclave were cooled to room temperature and analyzed by gas chromatography (internal standard method), the yield of methyl 2,3,5,6-tetrafluoro-4-hydroxymethyl benzoate was 20%. Also, dimethyl 2,3,5,6-tetrafluoroterephthalate recovery was 64%. Besides, methyl 2,3,5,6-tetrafluorobenzoate was produced as a by-product in 13%.

### Example 6

Into a 50 mL flask equipped with a reflux condenser, 200 mg of tris (triphenylphosphine) ruthenium dichloride and 50 mL of dichloromethane were charged. While stirring the obtained mixture at room temperature, to the mixture, a mixture of 45 mg of 6,6'-bis(aminomethyl)-1,2'-bipyridyl and 5 mL of dichloromethane was added, and immediately the color of the mixture was changed from bluish purple into black purple, and deposition of a crystal was found. The obtained mixture, per se, was stirred at room temperature for 30 minutes, and then the crystal was obtained by filtering the reaction mixture, followed by drying to yield 150 mg of a dark green powder containing a ruthenium complex. The melting point of the dark green powder was 196° to 200°C (decomposition).

### Example 7

Into a 100 mL autoclave equipped with a glass cylinder, 20 mg of the dark green powder obtained from Example 6, 15 mg of potassium hydroxide, 200 mg of dimethyl
2,3,5,6-tetrafluoroterephthalate and 10 g of toluene were charged. The inside of the autoclave was replaced with nitrogen and then replaced with hydrogen, followed by increasing the hydrogen pressure to 1.0 MPa. Then, upon increasing the temperature to 100°C, the inner pressure became 1.6 MPa. Upon stirring the contents of the autoclave at 170°C for 4 hours, the inner pressure became 1.5 MPa. When the contents of the autoclave were cooled to room temperature and analyzed by gas chromatography (internal standard method), the yield of methyl 2,3,5,6-tetrafluoro-4-hydroxymethyl benzoate was 21%. Also, dimethyl 2,3,5,6-tetrafluoroterephthalate recovery was 62%. Besides, methyl 2,3,5,6-tetrafluorobenzoate was produced as a by-product in 14%.

### Example 8

Into a 50 mL flask equipped with a reflux condenser, 200 mg of carbonyl (dihydride) tris (triphenylphosphine) ruthenium and 20 mL of tetrahydrofuran were charged. While stirring the obtained mixture at room temperature, to the mixture, a mixture of 63 mg of 2,6-bis[(phenylamino)methyl]pyridine and 5 mL of tetrahydrofuran was added, and then deposition of a crystal was found. The obtained mixture, per se, was stirred at room temperature for 30 minutes, and then the crystal was obtained by filtering the reaction mixture, followed by drying to yield 150 mg of a white pink powder containing a ruthenium complex. The melting point of the white pink powder was 183° to 186°C (decomposition).

### Example 9

Into a 100 mL autoclave equipped with a glass cylinder, 25 mg of the white pink powder obtained from Example 8, 200 mg of dimethyl 2,3,5,6-tetrafluoroterephthalate and 10 g of tetrahydrofuran were charged. The inside of the autoclave was replaced with nitrogen and then replaced with hydrogen, followed by increasing the hydrogen pressure to 1.0 MPa. Then, upon increasing the temperature to 130°C, the inner pressure became 1.5 MPa. Upon stirring the contents of the autoclave at 130°C for 4 hours, the inner pressure became 1.4 MPa. When the contents of the autoclave were cooled to room temperature and analyzed by gas chromatography (internal standard method), the yield of methyl 2,3,5,6-tetrafluoro-4-hydroxymethyl benzoate was 10%. Also, dimethyl 2,3,5,6-tetrafluoroterephthalate recovery was 88%. Besides, methyl 2,3,5,6-tetrafluorobenzoate was produced as a by-product in 0.1%.

### Example 10

Into a 50 mL flask equipped with a reflux condenser, 200 mg of carbonyl (dihydride) tris (triphenylphosphine) ruthenium and 20 mL of tetrahydrofuran were charged. While stirring the obtained mixture at room temperature, to the mixture, a mixture of 69 mg of N,N'-[bis(8-quinolyl)]ethane-1,2-diamine and 5 mL of tetrahydrofuran was added, and then deposition of a crystal was found. The obtained mixture, per se, was stirred at room temperature for 30 minutes, and then the crystal was obtained by filtering the reaction mixture, followed by drying to yield 150 mg of a grayish white powder containing a ruthenium complex. The melting point of the grayish white powder was 188° to 190°C (decomposition).

### Example 11

Into a 100 mL autoclave equipped with a glass cylinder, 13 mg of the grayish white powder obtained from Example 10, 200 mg of dimethyl 2,3,5,6-tetrafluoroterephthalate and 10 g of tetrahydrofuran were charged. The inside of the autoclave was replaced with nitrogen and then replaced with hydrogen, followed by increasing the hydrogen pressure to 1.0 MPa. Then, upon increasing the temperature to 130°C, the inner pressure became 1.5 MPa. Upon stirring the contents of the autoclave at 130°C for 4 hours, the inner pressure became 1.4 MPa. When the contents of the autoclave were cooled to room temperature and analyzed by gas chromatography (internal standard method), the yield of methyl 2,3,5,6-tetrafluoro-4-hydroxymethyl benzoate was 11%. Also, dimethyl 2,3,5,6-tetrafluoroterephthalate recovery was 85%. Besides, methyl 2,3,5,6-tetrafluorobenzoate was produced as a by-product in 0.1%.

### Example 12

Into a 50 mL flask equipped with a reflux condenser, 200 mg of carbonyl (dihydride) tris (triphenylphosphine) ruthenium and 20 mL of tetrahydrofuran were charged. While stirring the obtained mixture at room temperature, to the mixture, a mixture of 47 mg of 6,6'-bis(aminomethyl)-1,2'-bipyridyl and 5 mL of tetrahydrofuran was added, and then deposition of a crystal was found. The obtained mixture, per se, was stirred at room temperature for 30 minutes, and then the crystal was obtained by filtering the reaction mixture, followed by drying to yield 150 mg of a white yellow powder containing a ruthenium complex. The melting point of the white yellow powder was 185° to 189°C (decomposition).

### Example 13

Into a 100 mL autoclave equipped with a glass cylinder, 13 mg of the white yellow powder obtained from Example 12, 200 mg of dimethyl 2,3,5,6-tetrafluoroterephthalate and 10 g of tetrahydrofuran were charged. The inside of the autoclave was replaced with nitrogen and then replaced with hydrogen, followed by increasing the hydrogen pressure to 1.0 MPa. Then, upon increasing the temperature to 130°C, the inner pressure became 1.5 MPa. Upon stirring the contents of the autoclave at 130°C for 4 hours, the inner pressure became 1.4 MPa. When the contents of the autoclave were cooled to room temperature and analyzed by gas chromatography (internal standard method), the yield of methyl 2,3,5,6-tetrafluoro-4-hydroxymethyl benzoate was 9%. Also, dimethyl 2,3,5,6-tetrafluoroterephthalate recovery was 86%. Besides, methyl 2,3,5,6-tetrafluorobenzoate was produced as a by-product in 0.1%.

### Example 14

Into a 100 mL autoclave equipped with a glass cylinder, 22 mg of the black powder obtained from Example 4, 15 mg of potassium hydroxide, 65 mg of γ-butyrolactone and 10 g of toluene were charged. The inside of the autoclave was replaced with nitrogen and then replaced with hydrogen, followed by increasing the hydrogen pressure to 1.0 MPa. Then, upon increasing the temperature to 100°C, the inner pressure became 1.6 MPa. Upon stirring the contents of the autoclave at 170°C for 4 hours, the inner pressure became 1. 5 MPa. When the contents of the autoclave were cooled to approximately 25°C and analyzed by gas chromatography (internal standard method), the yield of 1,4-butanediol was 1%. Also, γ-butyrolactone recovery was 98%.

### Example 15

Into a 100 mL autoclave equipped with a glass cylinder, 13 mg of the grayish white powder obtained from Example 10, 65 mg of γ-butyrolactone and 10 g of tetrahydrofuran were charged. The inside of the autoclave was replaced with nitrogen and then replaced with hydrogen, followed by increasing the hydrogen pressure to 1.0 MPa. Then, upon increasing the temperature to 100°C, the inner pressure became 1.4 MPa. Upon stirring the contents of the autoclave at 130°C for 4 hours, the inner pressure became 1.3 MPa. When the contents of the autoclave were cooled to approximately 25°C and analyzed by gas chromatography (internal standard method), the yield of 1, 4-butanediol was 7%. Also, γ-butyrolactone recovery was 91%.

### Comparative Example 1

In Example 3, the same reactions as in Example 3 were performed except that 50 mg of
tris(triphenylphosphine)ruthenium dichloride was used in place of 50 mg of the reddish purple powder obtained from Example 1. When the contents of the autoclave were analyzed by gas chromatography (internal standard method), the generation of 2,3,5,6-tetrafluorobenzenedimethanol and the generation of methyl 2,3,5,6-tetrafluoro-4-hydroxymethyl benzoate were not found, and 2, 3, 5, 6-tetrafluoroterephthalic acid dimethyl ester recovery was 90%.

### Comparative Example 2

Into a 100 mL autoclave equipped with a glass cylinder, 20 mg of
bis{2-[bis(1,1-dimethylethyl)phosphino-κP]ethaneamine-κN}dic hlororuthenium (obtained from Aldrich), 20 mg of potassium hydroxide, 400 mg of dimethyl 2,3,5,6-tetrafluoroterephthalate and 20 g of toluene were charged. The inside of the autoclave was replaced with nitrogen and then replaced with hydrogen, followed by increasing the hydrogen pressure to 1.0 MPa. Then, upon increasing the temperature to 170°C, the inner pressure became 1.6 MPa. Upon stirring the contents of the autoclave at 170°C for 4 hours, the inner pressure became 1.5 MPa. When the contents of the autoclave were cooled to room temperature and analyzed by gas chromatography (internal standard method), the yield of 2,3,5,6-tetrafluorobenzenedimethanol was 8% and the yield of methyl 2,3,5,6-tetrafluoro-4-hydroxymethyl benzoate was 51%. Also, dimethyl 2,3,5,6-tetrafluoroterephthalate recovery was 22%.

### Comparative Example 3

Into a 100 mL autoclave equipped with a glass cylinder, 20 mg of a (p-cymene) ruthenium chloride dimer, 10 mg of sodium methoxide, 200 mg of dimethyl 2,3,5,6-tetrafluoroterephthalate and 10 g of tetrahydrofuran were charged. The inside of the autoclave was replaced with nitrogen and then replaced with hydrogen, followed by increasing the hydrogen pressure to 1.0 MPa. Then, upon increasing the temperature to 170°C, the inner pressure became 1.6 MPa. Upon stirring the contents of the autoclave at 170°C for 4 hours, the inner pressure became 1.6 MPa. When the contents of the autoclave were cooled to room temperature and analyzed by gas chromatography (internal standard method), the generation of
2,3,5,6-tetrafluorobenzenedimethanol and the generation of methyl 2,3,5,6-tetrafluoro-4-hydroxymethyl benzoate were not found, and mainly dimethyl 2, 3, 5, 6-tetrafluoroterephthalate was recovered. Besides, dimethyl 2,3,5-trifluoroterephthalate, methyl 2,3,5,6-tetrafluorobenzoate and the like were found to be produced as by-products.

### Comparative Example 4

Into a 100 mL autoclave equipped with a glass cylinder, 100 mg of 5% palladium/carbon, 200 mg of dimethyl 2,3,5,6-tetrafluoroterephthalate and 10 g of tetrahydrofuran were charged. The inside of the autoclave was replaced with nitrogen and then replaced with hydrogen, followed by increasing the hydrogen pressure to 1.0 MPa. Then, upon increasing the temperature to 170°C, the inner pressure became 1.6 MPa. Upon stirring the contents of the autoclave at 170°C for 4 hours, the inner pressure became 1.6 MPa. When the contents of the autoclave were cooled to room temperature and analyzed by gas chromatography (internal standard method), the generation of 2,3,5,6-tetrafluorobenzenedimethanol and the generation of methyl 2,3,5,6-tetrafluoro-4-hydroxymethyl benzoate were not found, and mainly dimethyl 2,3,5, 6-tetrafluoroterephthalate was recovered. Besides, dimethyl 2,3,5-trifluoroterephthalate, methyl 2,3,5,6-tetrafluorobenzoate and the like were found to be produced as by-products.

### INDUSTRIAL APPLICABILITY

According to the present invention, it is possible to obtain alcohol compounds useful in a variety of chemical products such as pharmaceutical and agrochemical ingredients and electronic materials, and as the synthetic intermediates thereof, and the like.

## Claims

1. A method for producing an alcohol compound, wherein a carboxylic acid ester compound is reduced with hydrogen in the presence of a ruthenium complex which is obtained by reacting a pyridine compound having at least one optionally substituted amino group with a ruthenium compound.

2. The production method according to claim 1, wherein in the pyridine compound, a pyridine ring is linked to the optionally substituted amino group through a linking group.

3. The production method according to claim 2, wherein the linking group is an optionally substituted alkylene group.

4. The production method according to claim 1, wherein the pyridine compound has two optionally substituted amino groups.

5. The production method according to claim 1, wherein the optionally substituted amino group is an amino group, an alkylamino group having 1 to 4 carbon atoms, a dialkylamino group having 2 to 8 carbon atoms, an arylamino group having 6 to 10 carbon atoms, a diarylamino group having 12 to 20 carbon atoms, or a cyclic amine group having 2 to 8 carbon atoms.

6. The production method according to claim 1, wherein the pyridine compound has a structure represented by the following formula 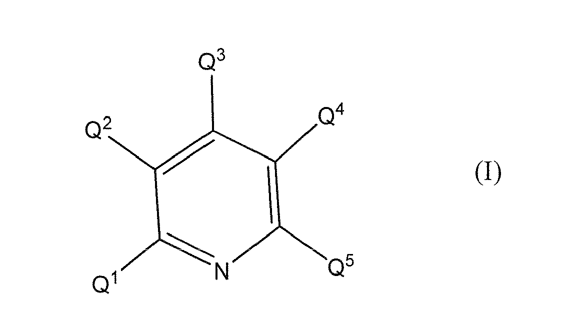 wherein Q¹, Q², Q³, Q⁴ and Q⁵ independently represent a hydrogen atom, an optionally substituted alkyl group, an optionally substituted aryl group, an optionally substituted alkoxy group, an amino group, or an optionally substituted aminoalkyl group, or each of Q¹ and Q², Q² and Q³, Q³ and Q⁴, and Q⁴ and Q⁵ may together represent a divalent group, with the proviso that at least one of Q¹, Q², Q³, Q⁴ and Q⁵ represents an amino group or an optionally substituted aminoalkyl group.

7. The production method according to claim 1, wherein the pyridine compound has a structure represented by the formula (A) 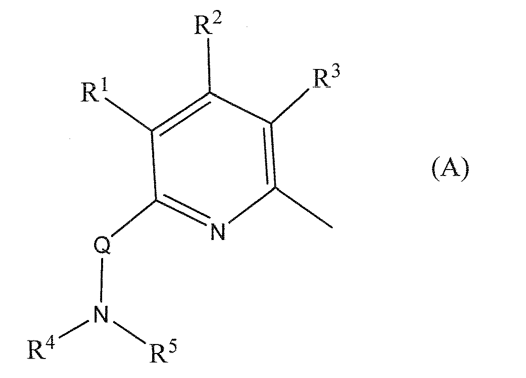 wherein R¹ represents a hydrogen atom, an optionally substituted alkyl group, an optionally substituted aryl group, or an optionally substituted alkoxy group, or with R² or Q, represents a divalent substituent; R² represents a hydrogen atom, an optionally substituted alkyl group, an optionally substituted aryl group, or an optionally substituted alkoxy group, or with R¹, represents a divalent substituent; R³ represents a hydrogen atom, an optionally substituted alkyl group, an optionally substituted aryl group, or an optionally substituted alkoxy group;
R⁴ and R⁵ independently represent a hydrogen atom, an optionally substituted alkyl group, or an optionally substituted aryl group, or R⁴ and R⁵ bind to each other, together with a nitrogen atom, to form a cyclic amino group having 2 to 8 carbon atoms; Q represents an optionally substituted alkylene group, or with R¹, represents a divalent substituent).

8. The production method according to claim 1, wherein the ruthenium compound is at least one member selected from the group consisting of a compound comprised of halogens and ruthenium, an aromatic compound-coordinated ruthenium dihalide dimer, a diene-coordinated ruthenium dihalide polymer, and a tris(triphenylphosphine)ruthenium compound.

9. The production method according to claim 1, wherein the carboxylic acid ester compound is a carboxylic acid ester compound having an aliphatic hydrocarbon group, a carboxylic acid ester compound having an aromatic hydrocarbon group, or a cyclic carboxylic acid ester compound.

10. The production method according to claim 1, wherein the carboxylic acid ester compound is a compound represented by the formula (6) 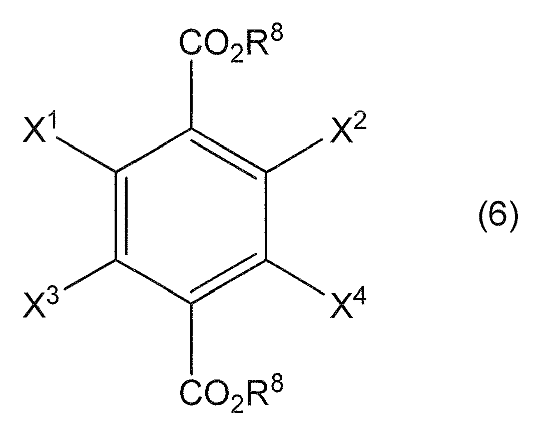 wherein R⁸ represents an alkyl group having 1 to 6 carbon atoms; and X¹, X², X³ and X⁴ each independently represent a hydrogen atom or a halogen atom, with the proviso that at least one of X¹, X², X³ and X⁴ is a halogen atom.

11. The production method according to claim 1, wherein the ruthenium complex is used in the range of 0.001 to 0.2 mol per mol of ester structure in the carboxylic acid ester compound.

12. The production method according to claim 1, wherein the carboxylic acid ester compound is reduced with hydrogen in the presence of a base.

13. A ruthenium complex which is obtained by reacting a pyridine compound having at least one optionally substituted amino group with a ruthenium compound.

14. A composition for producing an alcohol compound containing a ruthenium complex which is obtained by reacting a pyridine compound having at least one optionally substituted amino group with a ruthenium compound.
